# EUROPEAN PATENT SPECIFICATION

(11) **EP 3 518 868 B1**
(45) Date of publication and mention of the grant of the patent: **27.10.2021**
(21) Application number: 17778247.1
(22) Date of filing: 26.09.2017
(51) Int. Cl.: A61K 8/26, A61K 8/29, A61K 8/46, A61Q 11/00, A61K 8/81

(54) **ORAL CARE COMPOSITION**
MUNDPFLEGEZUSAMMENSETZUNG
COMPOSITION DE PROTECTION ORALE

(30) Priority: 29.09.2016 EP 16191589
(43) Date of publication of application: 07.08.2019
(73) Proprietor: Unilever IP Holdings B.V., 3013 AL Rotterdam (NL); Unilever Global IP Limited, Wirral, CH62 AZD (GB)
(72) Inventor: JOINER, Andrew, Wirral Merseyside CH63 3JW (GB); LITTLEWOOD, David, Thomas, Wirral Merseyside CH63 3JW (GB); DELFANTI, Cristina, 26841 Casalpusterlengo (IT); GUOLI, Angelica, 26841 Casalpusterlengo (IT); SALTELLI, Roberta, 26841 Casalpusterlengo (IT)
(74) Representative: Tansley, Sally Elizabeth
(86) International application number: PCT/EP2017/074412
(87) International publication number: WO 2018/060209

(56) References cited:
- WO-A1-2005/037127
- WO-A1-2013/070184
- WO-A1-2015/076817
- WO-A1-2016/097119
- DATABASE GNPD [Online] MINTEL; February 2009 (2009-02), "White Gloss", XP002764304, Database accession no. 1054267
- DATABASE GNPD [Online] MINTEL; July 2015 (2015-07), "Milky Flavour Toothpaste", XP002764305, Database accession no. 3284989

## Description

The present invention relates to an oral care composition that when coated onto the teeth aids tooth whitening and prevents staining.

### Background of the Invention

White teeth are seen as cosmetically attractive, however the enamel of the teeth can stain, for instance when certain foods or drinks are consumed. Hence, there is a need for products and devices to prevent/mitigate the staining of teeth.

### Background

WO2014205631 discloses an oral care composition containing (a) a zinc citrate; and (b) a surface-active organophosphate compound. The composition is said to prevent stain from depositing on a tooth surface or other oral surfaces.

Methods for reducing or preventing the staining capacity of a compound to a tooth are disclosed in WO14152829. The method includes the step of coating the tooth with a film comprising starch polymer or copolymer that is formed by crosslinking a starch.

WO 2013/070184 discloses dental whitening compositions, comprising an acrylate/octylacrylamide copolymer and one or more alkyl cellulose ethers. The compositions are said to prevent the teeth from staining.

Dental coating kits that are said to prevent teeth from staining are disclosed in EP1550428 and EP2854756.

With many tooth whitening products the teeth can appear coated with a film and the white shade of the teeth looks unnatural. There remains the need for a tooth whitening composition that gives the teeth a natural shade of white and adheres to the teeth in a manner such that the teeth do not look like they are coated. The compositions of the present invention when applied to the teeth give the teeth a naturally white appearance. Compositions according to the invention also mitigate tooth staining.

### Description of the Invention

The present invention relates a tooth coating and whitening composition comprising a whitening ingredient comprising materials with a high refractive index of at least 2.0 or blue dyes/pigments, an acryl based polymer and 5 to 25 wt% of the total composition of a smectite clay.

The invention further relates to the use of the above mentioned composition for tooth whitening and the prevention of staining.

### Detailed Description of the Invention

Compositions of the invention comprise a whitening agent. Suitable whitening agents include materials with a high refractive index or blue dyes/pigments.

In the context of the present invention a high refractive index is means materials with a refractive index of at least 2.0, more preferably at least 2.2, even more preferably at least 2.3, even more preferably still at least 2.4 and most preferably at least 2.5. The maximum refractive index of the material is not particularly limited but preferably the material has a refractive index up to 3.0, more preferably up to 2.9. Suitable materials to provide such a high refractive index are limited only in that they should be suitable for use in oral hygiene applications. Particularly suitable are metal salts and micas. Preferred are salts where the metal is selected from zinc (Zn), titanium (Ti) or a combination thereof. Additionally or alternatively the metal salt is (or at least comprises) a metal oxide. Especially preferred metal oxides are zinc oxide (ZnO), titanium dioxide (TiO₂), micas or a combination thereof. Most preferred, owing to their high refractive index and suitability for oral application are micas and titanium dioxide (TiO₂), especially preferred is mica coated with titanium dioxide.

Preferably the level of material with a high refractive index, more preferably a metal salt, in particular titanium dioxide and/or mica is from 0.1 to 8 wt% of the total composition, more preferably it is from 0.5 to 4 wt%.

The whitening agent can also be a blue colourant such as a pigment or blue dye, preferably it is a blue dye more preferably it is Patent blue.

Preferably, the level of blue colourant, more preferably blue pigment of blue dye is from 0.0005 wt% to 0.05 wt% of the total composition, more preferably from 0.0001 wt% to 0.01 wt%

Preferably, the composition comprises mixtures of whitening agents, more preferably a mica and a blue dye, most preferably the composition comprises mica coated with TiO₂ and patent blue.

Preferably, the weight ratio of any of the above mentioned combinations of metal salt/mica to blue colourant, more preferably titanium dioxide to blue dye, is from 2000:1 to 750:1, more preferably from 1500:1 to 1000:1.

Compositions according to the invention comprise a smectite clay, more preferable a montmorillonite clay.

Preferred smectite clays are selected from montmorillonites (bentonites, hectorites and derivatives thereof)(organically modified montmorillonite clays) such as quaternium-18 bentonite, quaternium-18 hectorite, stearalkonium bentonite and stearalkonium hectorite and mixtures thereof. Stearalkonium hectorite is particularly preferred.

Preferred compositions comprise from 5 to 25 wt% of the total composition of the above mentioned clay. More preferred compositions have 8 to 15 wt% of the total composition of the above mentioned clay.

The clay is believed to help the thickening of the product, in this way the extrusion from the container is facilitated and the dispersibility of the whitening material in particular the mica, in this way the appearance of the composition on the teeth look homogenous and natural.

Compositions of the invention comprise an acryl based polymer, more preferably an acrylate / octylacrylamide copolymer for example a copolymer of octylacrylamide (for example N-(1, 3,3-tetramethylbutyl)-propenamide) and one or more monomers selected from acrylic acid, methacrylic acid and their simple esters, in a particular embodiment, the acrylate / octylacrylamide copolymer is 2-propenoic acid, 2-methyl- 2-methylpropyl ester, polymer with 2-propenoic acid and N-(1,1,3,3 -tetramethylbutyl)-2-propenamide (example DERMACRYL 79 (commercially available from National Starch or Akzo Nobel).

Preferably the level of acryl based polymer is from 2 to 20 Wt % of the total composition, more preferably from 5 to 15 wt% of the total composition.

Preferably which the weight ratio of clay to acryl based polymer is from 1.3 :1 to 1:1 Compositions according to the invention will usually contain a continuous phase, which may comprise water and/or alcohol more preferably a polyhydric alcohol. The amount of alcohol, preferably polyhydric alcohol generally ranges from 20 to 85 wt% (based on the total weight of the composition), preferably the level of water is 7 wt% or below and the level of alcohol is from 30 wt%. to 75 wt% of the total composition.

A preferred alcohol for use in the present invention and at the present invention is isopropyl alcohol.

In a preferred embodiment the composition comprises an ester in the continuous phase, more preferably ethyl acetate. Preferably the level of ethyl acetate is form 10 to 30 wt% of the total composition more preferably form 15 to 25 wt%.

The oral care composition of the present invention may contain a variety of other ingredients which are common in the art to enhance physical properties and performance. These ingredients include antimicrobial agents, anti-inflammatory agents, anti-caries agents, plaque buffers, fluoride sources, vitamins, plant extracts, desensitizing agents, anti- calculus agents, biomolecules, flavours, proteinaceous materials, preservatives, opacifying agents, micas, colouring agents, pH-adjusting agents, sweetening agents, particulate abrasive materials, polymeric compounds, buffers and salts to buffer the pH and ionic strength of the compositions, and mixtures thereof. Such ingredients typically and collectively makeup less than 20 percent by weight of the composition, and preferably, from 0.0 to 15 percent by weight, and most preferably, from 0.01 to 12 percent by weight of the composition, including all ranges subsumed therein.

It is highly preferable if compositions of the invention are orally acceptable. By "orally acceptable" it is meant that the compositions are safe for use in the mouth at the levels required.

Example of the invention will now be illustrated by the following non-limiting examples:

**Table 1**

| Trade Name | INCI name | Examp Wt le 1 % | Exam W ple 2 t% | Example A |
|---|---|---|---|---|
| Dermacryl 79 | Acrylate/Octylacrylamide | 10 | 15 | - |
| Eudragit RL | copolymer of ethyl acrylate, methyl methacrylate methacrylic acid ester with quaternary ammonium groups | | | 7 |
| Bentone gel TN V | C12-15 alkyl benzoate, stearalkonium hectorie, propylene carbonite | 12 | 12 | - |
| | Titanium dioxide | 1.5 | 1.5 | 1.0 |
| | Ethyl acetate | 20 | 20 | |
| Patent Blue Patent | C l 42051 | 0.00125 | 0.00125 | 0.00125 |
| | Titanium Dioxide | 1.5 | 1.5 | 1.0 |
| Ethyl cellulose | | - | - | 4.0 |
| PEG -3000 | | - | - | 2.0 |
| | Isopropyl alcohol and minor | To 100 | To 100 | To 100 |

Examples 1 and 2 when coated on the teeth enhanced the white appearance and yet the enhancement did not look unnatural nor did the teeth appear coated

The following Examples were applied to enamel bovine chips and were tested against staining solutions prepared from Black tea. Delata E was calculated. 12 measurements were made per calculation and the average calculated.

The results are presented in Table 2

**Table 2:**

| | Exam ple 1 | Example 2 | E example A |
|---|---|---|---|
| ΔE | 2.46 | 3.81 | 13.52 |

The results of table 2 demonstrate that Examples of the invention prevent the staining of the teeth.

The following compositions were prepared.

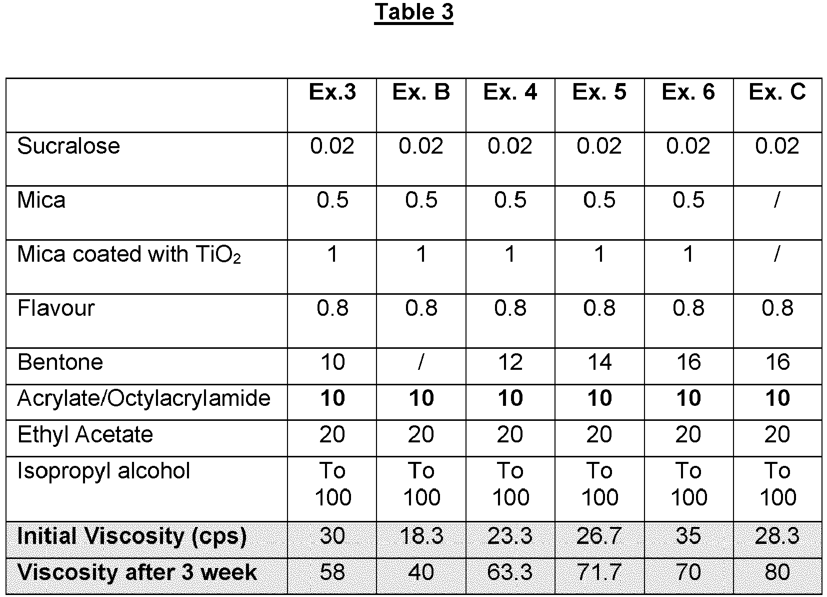

The sedimentation of the above Examples was measured and is recorded in Table 4 below

**Table 4**

| | %SEDIMENT | %BENTONE |
|---|---|---|
| Example B | 1.822% | 0 |
| Example 3 | 2.468% | 10 |
| Example 4 | 2.3911% | 12 |
| Example 5 | 2.74495% | 14 |
| Example 6 | 3.06480% | 16 |
| Example C | 0.97921% | 16 |

Examples according to the invention coated the teeth with a natural looking film yet had acceptable sedimentation and viscosity.

## Claims

1. A tooth coating and whitening composition comprising a whitening ingredient comprising materials with a high refractive index of at least 2.0 or blue dyes/pigments, an acryl based polymer and 5 to 25 wt% of the total composition of a smectite clay.

2. A composition according to claim 1 in which the acryl based polymer comprises an acrylate / octylacrylamide copolymer.

3. A composition according to claim 1 or claim 2 in which the acryl based polymer comprises 2-propenoic acid, 2-methyl-, 2-methylpropyl ester, polymer with 2-propenoic and N-(1,1,3,3-tetramethylbutyl)-2-propenamide.

4. A composition according to any preceding claim in which the level of acryl based polymer is from 5 to 15wt% of the total composition.

5. A composition according to any preceding claim in which the clay comprises stearalkonium hectorite.

6. A composition according to any preceding claim in which the level of clay is from 8 to 15 wt% of the total composition.

7. A composition according to any preceding claim in which the weight ratio of clay to acryl based polymer is from 1.3 :1 to 1:1.

8. A composition according to any preceding claim in which the whitening ingredient comprises titanium dioxide and/or mica.

9. A composition according to claim 8 in which the level of titanium dioxide and/or mica is from 0.5 to 4 wt% of the total composition.

10. A composition according to any preceding claim in which the whitening ingredient comprises mica and a blue dye.

11. A composition according to any one of claims 8 to 10 in which the weight ratio of titanium dioxide to blue dye, is from 2000:1 to 750:1.

12. A composition according to any preceding claim which comprises from 30 to 75 wt% of the total composition of an alcohol.

13. A composition according to any preceding claim which comprises ethyl acetate.

14. Use of a composition according to any preceding claim for tooth whitening .

15. Use of a composition according to any preceding claim for the prevention of tooth staining.

## Patentansprüche

1. Zahnbeschichtungs- und Weißungszusammensetzung, umfassend einen Weißungsbestandteil, umfassend Materialien mit einem hohen Brechungsindex von mindestens 2,0 oder blaue Farbstoffe/Pigmente, ein Polymer auf Acrylbasis und 5 bis 25 Gew.-% der gesamten Zusammensetzung von einem Smektitton.

2. Zusammensetzung nach Anspruch 1, bei der das Polymer auf Acrylbasis ein Acrylat/Octylacrylamid-Copolymer umfasst.

3. Zusammensetzung nach Anspruch 1 oder Anspruch 2, bei der das Polymer auf Acrylbasis 2-Propensäure, 2-methyl-, 2-methylpropylester, Polymer mit 2-Propen und N-(1,1,3,3-Tetramethylbutyl)-2-propenamid umfasst.

4. Zusammensetzung nach irgendeinem vorhergehenden Anspruch, bei der der Gehalt an Polymer auf Acrylbasis 5 bis 15 Gew.-% der gesamten Zusammensetzung beträgt.

5. Zusammensetzung nach irgendeinem vorhergehenden Anspruch, bei der der Ton Stearalkoniumhectorit umfasst.

6. Zusammensetzung nach irgendeinem vorhergehenden Anspruch, bei der der Tongehalt 8 bis 15 Gew.-% der gesamten Zusammensetzung beträgt.

7. Zusammensetzung nach irgendeinem vorhergehenden Anspruch, bei der das Gewichtsverhältnis von Ton zu Polymer auf Acrylbasis 1,3:1 bis 1:1 beträgt.

8. Zusammensetzung nach irgendeinem vorhergehenden Anspruch, in der der Weißungsbestandteil Titandioxid und/oder Glimmer umfasst.

9. Zusammensetzung nach Anspruch 8, bei der der Gehalt an Titandioxid und/oder Glimmer 0,5 bis 4 Gew.-% der gesamten Zusammensetzung beträgt.

10. Zusammensetzung nach irgendeinem vorhergehenden Anspruch, bei der der Weißungsbestandteil Glimmer und einen blauen Farbstoff umfasst.

11. Zusammensetzung nach irgendeinem der Ansprüche 8 bis 10, bei der das Gewichtsverhältnis von Titandioxid zu blauem Farbstoff 2000:1 bis 750:1 beträgt.

12. Zusammensetzung nach irgendeinem vorhergehenden Anspruch, die 30 bis 75 Gew.-% der gesamten Zusammensetzung von einem Alkohol umfasst.

13. Zusammensetzung nach irgendeinem vorhergehenden Anspruch, die Ethylacetat umfasst.

14. Verwendung einer Zusammensetzung nach irgendeinem vorhergehenden Anspruch zur Zahnweißung.

15. Verwendung einer Zusammensetzung nach irgendeinem vorhergehenden Anspruch zur Verhinderung von Zahnverfärbung.

## Revendications

1. Composition de revêtement et blanchiment de dent comprenant un ingrédient blanchissant comprenant des matériaux avec un indice de réfraction élevé d'au moins 2,0 ou des colorants/pigments bleus, un polymère à base d'acryle et de 5 à 25 % en masse de la composition totale d'une argile de smectite.

2. Composition selon la revendication 1, dans laquelle le polymère à base d'acryle comprend un copolymère d'acrylate/ octylacrylamide.

3. Composition selon la revendication 1 ou revendication 2, dans laquelle le polymère à base d'acryle comprend de l'acide 2-propénoïque, ester 2-méthyl-, 2-méthylpropylique, polymère avec 2-propénoïque et N-(l,l,3,3-tétraméthylbutyl)-2-propénamide.

4. Composition selon l'une quelconque des revendications précédentes, dans laquelle la teneur en polymère à base d'acryle est de 5 à 15 % en masse de la composition totale.

5. Composition selon l'une quelconque des revendications précédentes, dans laquelle l'argile comprend de l'hectorite de stéaralkonium.

6. Composition selon l'une quelconque des revendications précédentes, dans laquelle la teneur en argile est de 8 à 15 % en masse de la composition totale.

7. Composition selon l'une quelconque des revendications précédentes, dans laquelle le rapport de masse d'argile à polymère à base d'acryle est de 1,3:1 à 1:1.

8. Composition selon l'une quelconque des revendications précédentes, dans laquelle l'ingrédient blanchissant comprend du dioxyde de titane et/ou mica.

9. Composition selon la revendication 8, dans laquelle la teneur en dioxyde de titane et/ou mica est de 0,5 à 4 % en masse de la composition totale.

10. Composition selon l'une quelconque des revendications précédentes, dans laquelle l'ingrédient blanchissant comprend du mica et un colorant bleu.

11. Composition selon l'une quelconque des revendications 8 à 10, dans laquelle le rapport massique de dioxyde de titane à colorant bleu, est de 2 000:1 à 750:1.

12. Composition selon l'une quelconque des revendications précédentes qui comprend de 30 à 75 % en masse de la composition totale d'un alcool.

13. Composition selon l'une quelconque des revendications précédentes qui comprend de l'acétate d'éthyle.

14. Utilisation d'une composition selon l'une quelconque des revendications précédentes pour le blanchiment de dent.

15. Utilisation d'une composition selon l'une quelconque des revendications précédentes pour la prévention de coloration de dent.
